# EUROPEAN PATENT APPLICATION

(11) **EP 2 143 784 A1**
(43) Date of publication of application: **13.01.2010**
(21) Application number: 08720640.5
(22) Date of filing: 27.03.2008
(51) Int. Cl.: C12N 1/20, A61K 35/74, A61P 37/08

(54) **COMPOSITION COMPRISING LACTIC ACID BACTERIUM HAVING HIGH ANTI-ALLERGIC ACTIVITY, AND METHOD FOR PRODUCTION OF THE LACTIC ACID BACTERIUM**

(30) Priority: 30.03.2007 JP 2007090476
(71) Applicant: Kirin Holdings Kabushiki Kaisha, Tokyo 104-8288 (JP)
(72) Inventor: NISHIDA, Takeo, Yokohama-shi Kanagawa 236-0004 (JP); AWAYAMA, Hitoshi, Gunma 370-1295 (JP)
(74) Representative: HOFFMANN EITLE
(86) International application number: PCT/JP2008/000761
(87) International publication number: WO 2008/129841

(57) **Abstract**

It is to provide a lactic acid bacterium having a high antiallergic activity and stable antiallergic activity that can be used effectively in various product forms and in various use applications. By conducting a heat treatment at 60C or more and less than 100°C for 10 minutes or more and less than 60 minutes to *Lactobacillus paracasei* KW3110 strain or its mutant strain, the antiallergic activity of the lactic acid bacterium can be significantly increased to produce *Lactobacillus paracasei* KW3110 strain and its mutant strain having a stable antiallergic activity and high antiallergic activity. According to the present invention, an antiallergic composition comprising a lactic acid bacterial cell which antiallergic activity of the lactic acid bacterial cell has been significantly increased by the heat treatment of the present invention, or having a stable high antiallergic activity can be provided.

## Description

### Technical Field

The present invention relates to a lactic acid bacterium composition with high antiallergic activity, a method for producing the lactic acid bacterium, particularly, a method for producing a lactic acid bacterial cell with high antiallergic activity, comprising heat treating a lactic acid bacterium with excellent antiallergic activity, *Lactobacillus paracasei* KW3110 strain or its mutant strain, to confer a high antiallergic activity and to confer a stable antiallergic activity, and to an antiallergic composition comprising the lactic acid bacterial cell with high antiallergic activity as an active ingredient.

### Background Art

Allergy is one of the diseases most frequently occurred in the developed countries. The development mechanism of allergy is generally classified into four classes, that is type I to type IV. Type I allergy associated with IgE antibodies are typified by hay fever, asthma, urticaria, anaphylaxy shock and the like. Type II allergy associated with IgG antibodies and IgM antibodies is a cytotoxic reaction which activates complement systems, and fetal erythroblastosis and autoimmune hemolytic anemia belong to this type. Type III allergy is a tissue injury caused by antigen-antibody complex, and is a reaction typified by Alex reaction, serum sickness, glomerulonephritis and the like. Type IV allergy is a delayed allergy (delayed hypersensitivity) associated with T cells, typified by tuberculin reaction or contact dermatitis. It is said that types I, II and IV among allergy are associated with the development of food allergy. On the other hand, it is known that environmental allergy, that is hay fever, atopic dermatitis, bronchial asthma, chronic allergic rhinitis, allergic conjunctivitis and the like have mainly the development mechanism of type I.

Type I allergy mentioned above is characterized by induction of allergen-specific IgE, release of chemical mediators such as histamine, leukotriene and the like. Immune response occurs from interaction of various cells, and helper T cells (Th) are one of the types of cells involved in this reaction. Helper T cells being classified into subgroups of T cells, generate various cytokines (helper factors) by recognizing antigens, and control the induction of immune response. Helper T cells are classified into Th1 cells and Th 2 cells based on their cytokine producing ability. Th2 cytokines such as IL-4, IL-5, IL-13 and the like are necessary, so that class switch of antibody occurs in B cells to generate IgE antibodies. Actually, it is known that Th2 cells are increased in lymphocytes of allergic patients. In other words, allergens that have penetrated from the outer world, are presented to T cells, in a conditions that a part is bound to MHC class II molecules by antigen presenting cells such as dendritic cells, macrophages and the like, and then Th2 cells are activated and differentiated. Th2 cytokines released by Th2 cells induce class switch of B cells, IgE antibodies are generated, and the IgE antibodies are bound with mast cells in the tissues and FcR on the surface of basophils in blood. Allergens are recognized by IgE antibodies bound to mast cells or on the surface of basophils at the time of the next invasion, and crosslinking are formed between IgE antibodies. This stimulation as a trigger, mast cells and basophils release significantly the chemical mediators, and various symptoms of allergy appear.

As for prevention and treatment of allergy through IgE or chemical mediators, examples include: antihistamine that inhibit signaling from the peripheral nerve by binding in an antagonistic manner with histamine to histamine receptor; antiallergic drug for trying to relief symptoms by diminishing the activity of chemical mediators-producing cells; steroid relieving inflammation by diminishing immune response; hyposensitization therapy inducing tolerance by injecting periodically the allergen itself. However, side effects are being problems for all of these, and none of these have definitive effects.

Recently, a method for controlling the production of Th2 cytokine is getting attention for the purpose of suppressing IgE. Certain bacterium such as *tubercle bacilli,* hemolytic *streptococcus,* the lactic acid bacteria and the like have been reported to enhance Th1 immunity and suppress Th2 immunity to lower IgE as a result (Clinical Immunology, vol. 32, p. 454, 1999; International Archives of Allergy and Immunology, vol. 115, p. 278, 1998; Japanese Laid-Open Patent Application No.9-2959).

Among these, the lactic acid bacteria being easy to apply to foods from the point of safety, are useful materials. However, as for the lactic acid bacteria, it is not appropriate to say that all lactic acid bacteria have strong effect to enhance Th1 immunity, and it is indispensable to select useful bacterial strains. As for antiallergic lactic acid bacteria, *L. rhamnosus* LGG strain is publicly known and it is reported that by administering to expectant mothers, the development of atopic dermatitis of the child is suppressed (Lancet, vol. 357, p. 1076, 2001). The use of the lactic acid bacteria as antiallergical drugs is disclosed in several Patent Gazettes. For example, Japanese Laid-Open Patent Application No. 9-2959 discloses the use of the lactic acid bacteria as antiallergic drug wherein IgE production level is 30 ng/ml or less when the lactic acid bacteria such as *L. acidophilus, L. brevis, L. buchnerii, L. casei* and the like are added to culture mouse lymphocytes; Japanese Laid-Open Patent Application No. 10-309178 discloses the use of bifidobacteria such as *Bifidobacterium. infantis, Bifidobacterium. breve, Bifidobacterium. longum, Bifidobacterium. bifidum* and the like as antiallergic drug to treat especially food allergy; and Japanese Laid-Open Patent Application No. 2000-95697, the use of lacid acid bacteria such as *Enterococcus faecalis, Lactobacillus leuteri* and the like, as inhibitor of type I allergy such as allergic bronchial asthma, chronic allergic rhinitis, atopic dermatitis and the like.

On the other hand, it is known that the enhancement of Th1 immunity by the lactic acid bacteria and the like relates to the activation of cellular immunity such as macrophage, killer T cells, NK cells through the production of IL12, IFN.γ and it is known that it leads to the resistance to viral or bacterial infection, or development of cancer. Specifically, IL-12 produced by macrophage, leads to the resistance against foreign enemy, cancer through differentiation of the naive helper T cells to Th1 cells, activation of monocytes, macrophage or NK cells. Therefore, it is suggested that the lactic acid bacteria strain being able to induce strong IL-12 production can be used as immunoadjuvant (Cancer Immunology Immunotherapy, vol. 49, p. 157, 2000; Japanese Laid-Open Patent Application No. 7-228536, Japanese Laid-Open Patent Application No. 2002-80364).

Recently, there are many people whose constitution of their body has changed, such as atopic dermatitis or hay fever, and it seems to be caused by allergy. This is becoming a social problem. Among these persons, treatment using various drugs is provided to patients having particularly severe symptoms. However, most of the patients have not reached the point to receive full-scale treatment, and as actual situation, they are receiving symptomatic therapy to keep daily life, including folk remedy.

In view of this situation, recently, many researches are conducted actively targeting to suppress allergy by diet. As a result, antiallergic effects are found in ingredients of various foods or drinks such as Japanese basil oil, fish oil, particular tea polyphenol and the like, and treatment by using these are provided.

As ingredients of foods and beverages having antiallergic effect, similar effects in particular lactic acid bacteria are now known such as described above, and some yogurts or the lactic acid bacteria beverages using the lactic acid bacteria are practically used as foods or drinks having antiallergic effect. As for prevention or treatment using this kind of antiallergic foods and drinks, it is possible to ingest under safe and mild conditions but it is necessary to ingest these every day continuously, and it is also necessary to be effective with the amount possible to ingest continuously. However, conventionally, since a method for obtaining and selecting microbial strains with high antiallergic activity has not been clarified, any promising microbial strain was not obtained, thus it was not a satisfactory method for practical use.

Recently, the present inventors have found out a lactic acid bacterium showing interleukin 12 production level of a particular level or more, and interleukin 4 production level being less than a particular level, and developed and disclosed an antiallergic composition having an excellent antiallergic activity consisting of the lactic acid bacterium (Japanese Laid-Open Patent Application No. 2005-139160). Examples of such lactic acid bacteria include *Lactobacillus paracasei* KW3110 strain, *Lactobacillus plantarum* KW4110 strain, *Lactobacillus paracasei* KW3925 strain, *Lactobacillus paracasei* KW3926 strain, and *Streptococcus salivarius* KW3210 strain.
Further, at the same time, they developed and disclosed foods or drinks having an excellent antiallergic activity comprising *Lactobacillus paracasei* KW3110 strain and its mutant strain as an active ingredient, as a lactic acid bacterium having a particularly excellent antiallergic activity among the lactic acid bacteria (Japanese Laid-Open Patent Application No. 2005-137357; Japanese Patent No. 3585487).

Thereafter, the following has been reported concerning the antiallergic effect of *Lactobacillus paracasei* KW3110 strain. For example, in an *in vitro* system experiment, *Lactobacillus paracasei* KW3110 strain was selected as having the strongest ability to induce IL-12 production and to suppress IL-4 from about 100 strains of lactic acid bacteria. Using the selected KW3110 strain, an *in vivo* experiment was carried out to a mouse being allergic-sensitized with OVA, and antiallergic effects comprising IgE suppression in blood, IL-12 production and IL-4 suppression tendencies were reported to be observed (Int. Arch. Allergy Immunol.,135, 205, 2004). Further, it has been reported that by preparing an yoghurt with KW3110 strain having an antiallergic effect, or *Lactobacillus delbrueckii* B strain not having such a high antiallergic activity, and conducting an experiment using theses yoghurts in human, the yoghurt prepared with KW3110 strain has been confirmed to be associated with Th2 cell proliferation and suppression of eosinophil activity (Allergology In. 54, 143, 2005).

As it is stated in the above, among various known lactic acid bacteria having an antiallergic activity, *Lactobacillus paracasei* KW3110 strain has a particularly excellent antiallergic effect. However, when using the lactic acid bacterium as an active ingredient in various product forms and in various use, further enhancement of antiallergic activity of the active ingredient, and the development of a more stable antiallergic active ingredient are awaited in order to further enhance its affectivity.

Patent Document 1: Japanese Laid-Open Patent Application No. 7-228536
Patent Document 2: Japanese Laid-Open Patent Application No. 9-2959
Patent Document 3: Japanese Laid-Open Patent Application No. 10-309178
Patent Document 4: Japanese Laid-Open Patent Application No. 2000-95697
Patent Document 5: Japanese Laid-Open Patent Application No. 2002-80364
Patent Document 6: Japanese Laid-Open Patent Application No. 2005-137357
Patent Document 7: Japanese Laid-Open Patent Application No. 2005-139160
Patent Document 8: Japanese Patent No. 3585487
Non-Patent Document 1: Rinsho Meneki (Clinical Immunology) 1999, vol.32, p. 454
Non-Patent Document 2: International Archives of Allergy and Immunology, 1998, vol. 115, p.278
Non-Patent Document 3: Lancett, 2001, vol. 357, p. 1076
Non-Patent Document 4: Cancer Immunology Immunotherapy, 2000, vol.49, p.157
Non-Patent Document 5: Int. Arch. Allergy Immunol., 135, 205, 2004
Non-Patent Document 6: Allergology Int. 54, 143, 2005

### Disclosure of the Invention

### Object to be solved by the present invention

The object of the present invention is to enhance the antiallergic activity of the active ingredient when using lactic acid bacteria having an antiallergic activity in various product forms and in various use, and to provide a lactic acid bacterium having a more stable antiallergic activity, in order to further enhance its effectiveness.

### Means to solve the object

The present inventors made a keen study to solve the above object on active ingredients having a high antiallergic activity consisting of lactic acid bacteria. They found out that by conducting a heat treatment on the lactic acid bacterium having an excellent antiallergic activity, *Lactobacillus paracasei* KW3110 strain or its mutant strain, within a particular temperature range and for a particular time period, the antiallergic activity can be significantly increased, and by stopping the activity of the lactic acid bacterium itself, and preventing as much as possible the denaturation of the bacterium itself by heat treatment, an active ingredient having a stable and high antiallergic activity can be obtained. The present invention has been thus completed.

Specifically, the present invention relates to a method for producing *Lactobacillus paracasei* KW3110 strain or its mutant strain having a high antiallergic activity with a stable antiallergic activity, by conducting heat treatment to *Lactobacillus paracasei* KW3110 strain or its mutant strain at a temperature of 60°C or more and less than 100°C, for a time period of 10 minutes or more and less than 60 minutes, to significantly increase the antiallergic activity of the lactic acid bacterium. Further, the present invention encompasses a method conducting heat treatment to *Lactobacillus paracasei* KW3110 strain or its mutant strain, after culturing *Lactobacillus paracasei* KW3110 strain or its mutant strain, and removing medium components by conducting washing treatment to the cultured bacterial cell, particularly conducting an effective heat treatment to obtain a lactic acid bacterial cell having a stable and high antiallergic activity.

Further, the present invention relates to an antiallergic composition which effectiveness has been further enhanced when using in various product forms and in various use, comprising as an active ingredient a lactic acid bacterial cell having a stable and high antiallergic activity, consisting of *Lactobacillus paracasei* KW3110 strain or its mutant strain produced by the production method of the present invention.

Specifically, the present invention relates to (1) a method for producing a heat treated bacterial cell of *Lactobacillus paracasei* KW3110 strain or its mutant strain having a high antiallergic activity, wherein a heat treatment is conducted to *Lactobacillus paracasei* KW3110 strain or its mutant strain at a temperature of 60°C or more and less than 100°C, for a time period of 10 minutes or more and less than 60 minutes; (2) the method for producing a heat treated bacterial cell of *Lactobacillus paracasei* KW3110 strain or its mutant strain having a high antiallergic activity according to (1), wherein the heat treatment to *Lactobacillus paracasei* KW3110 strain or its mutant strain is conducted after culturing *Lactobacillus paracasei* KW3110 strain or its mutant strain, and removing a medium component by conducting washing treatment to the cultured bacterium; (3) a heat treated bacterial cell of *Lactobacillus paracasei* KW3110 strain or its mutant strain produced by the production method of (1) or (2); (4) an antiallergic composition comprising the heat treated bacterial cell of *Lactobacillus paracasei* KW3110 strain or its mutant strain produced by the production method of (1) or (2) as an active ingredient.

### Brief Explanation of the Drawings

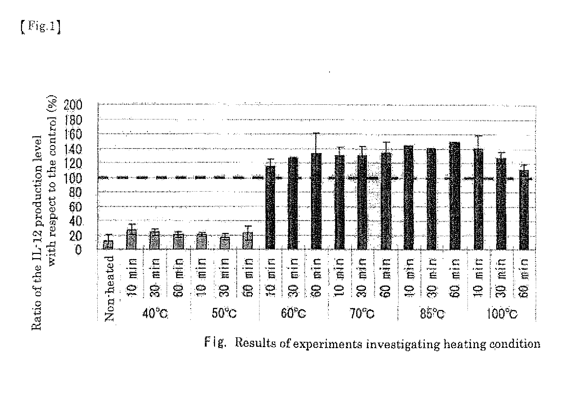

[Fig. 1] It is a figure showing the IL-12 production level ratio (ratio with respect to the control) for each heating condition in an experiment measuring the antiallergic activity for the heating condition of the lactic acid bacterium of the present invention, in the Examples of the present invention.

### Best Mode for Carrying Out the Invention

The present invention relates to conducting a heat treatment to *Lactobacillus paracasei* KW3110 strain or its mutant strain having an excellent antiallergic activity at a temperature of 60°C or more and less than 100°C, for a time period of 10 minutes or more and less than 60 minutes, to significantly increase the antiallergic activity of the lactic acid bacteria and to produce lactic acid bacterium having a stable and high antiallergic activity.

### (Lactic acid bacterium used in the present invention)

As for the lactic acid bacteria with high antiallergic activity used as active ingredients in the present invention, for example *Lactobacillus paracasei* (*L. paracasei*) KW3110 strain, can be obtained from Japan Dairy Technical Association as *L. casei* L14 strain. Meanwhile, though there is a statement of Japan Dairy Technical Association that L14 strain is *L. casei,* when the present inventors have analyzed by using RFLP (Restriction Flagment Length Polymorphism) and AFLP (Amplified Flagment Length Polymorphism) by using RiboPrinter (QUALICON), the strain was determined to be *L. paracasei,* therefore it is stated as *L. paracasei* in the present invention. *L. paracasei* KW3110 used as active ingredients of the antiallergic composition in the present invention can be obtained from Japan Dairy Technical Association as described in the above, and moreover, it is deposited as FERM BP-08634 at the National Institute of Advanced Industrial Science and Technology, International Patent Organism Depositary, which is an International Depositary Authority, according to Budapest Treaty on the deposit of microorganism for patent procedure.

The lactic acid bacteria being the active ingredients of the present invention can be used as active ingredients of the composition of the present invention, by cultured and proliferated appropriately in a medium for culturing the lactic acid bacteria known to a skilled person such as M. R. S. (de Man, Rogosa, Sharpe) medium and the like, conducting heat treatment according to the present invention, powdered by lyophilizing or by spray-drying with a spray according to need to be used. Furthermore, for process management, or as for a method for measuring the antiallergic activity of the produced lactic acid bacterial cell , it is possible to measure by using the method for measuring the antiallergic activity of the lactic acid bacteria wherein the level of Th1-inducing cytokine and/or Th2-inducing cytokine generated by suspending lymphocytes derived from animal spleen sensitized with allergen in a medium containing the allergen, and cultured by adding the lactic acid bacteria to be tested.

The mutant strain of *L. paracasei* KW3110 strain used in the present invention can be prepared by mutating the lactic acid bacterium and preparing a mutant strain with a high antiallergic activity for use. As for a means to raise mutation to bacterial cell, it can be carried out by a publicly known mutating means such as UV and the like. For example, by mutating L. paracasei KW3110, and by using a method for estimating antiallergic activity of the lactic acid bacteria described in the present specification, it is possible to obtain mutant strain with increased antiallergic activity and make use of these.

### (Selection of derivative strains)

In the present invention, it is possible to select strain with different characteristics occurred from the lactic acid bacteria strain obtained in the present invention as derivative strain and to make use of these. For example, L. paracasei KW3110 strain is cultured by a static culture until it reaches a prescribed number of bacteria, with the use of a medium such as MRS medium, at a prescribed temperature; diluting the culture solution in a fresh MRS medium; suspending 10% of the microbial suspension to PBS (tablet from Dainippoin Pharmaceutical dissolved at a designated concentration) wherein pH of PBS was adjusted to 3.0 with hydrochloric acid; and incubating at 37°C for 3 hours. The microbial suspension treated with acid is diluted with PBS, poured in MRS plate medium, to form colonies. The colonies formed are selected by taking the color tone and the like as index, and further, one strain is selected among these colonies selected based on the color tone, and cultured in a MRS medium for 48 hours. The antiallergic activity of the culture is measured with the method for measuring the antiallergic activity of the present invention described in Example 1, and the antiallergic activity of each colony is determined. By using this method, the microbial strains with high antiallergic activity are selected and obtained from the strains tested. In the Example of the present invention, strains separated as derivative strain of KW3110 was named No. 90 strain, and are deposited at the National Institute of Advanced Industrial Science and Technology, International Patent Organism Depositary, which is an International Depositary Authority as FERM BP-08635, according to Budapest Treaty on the deposit of microorganism for patent procedure.

### (Antiallergic activity of the lactic acid bacteria produced by the present invention)

When IgE antibodies are generated in response to antigenic stimulation, the IgE antibodies bind to Fc receptors on the surface of the mast cells in the tissues or on the surface of basophils in blood, then recognized by IgE antibodies bond on the surface of mast cells or on the basophils surface upon the secondary antigenic stimulation (re-invasion of allergen), and crosslinking are formed between the IgE antibodies, and when mast cells or basophils release vast amounts of chemical mediators with this stimulation as a trigger, then various symptoms of allergy appear. Therefore, it is necessary to suppress IgE for treating and preventing allergy, and for that purpose, to enhance Th1 immunity to suppress Th2 immunity. The lactic acid bacteria of the present invention induce strongly interleukin 12 (IL-12) production being the index of Th1 immunity, and at the same time, suppress strongly the interleukin 4 (IL-4) production being the index of Th2 immunity, in an *in vitro* system using mouse lymphocytes. Therefore, the lactic acid bacteria of the present invention have effects for treating and preventing allergy based on the acting mechanism that the production of IgE antibody is suppressed by enhancing Th1 immunity and suppressing Th2 immunity.

### (Heat treatment of the present invention)

In the present invention, *L. paracasei* KW3110 strain or its mutant strain is subjected to heat treatment at a certain temperature for a certain time period, to increase its antiallergic activity. As heating temperature, a temperature of 60°C or more and less than 100°C is used, and as heating time, a time of 10 minutes or more and less than 60 minutes is applied. Particularly, a temperature of 60 to 85°C is preferred, and more preferably a temperature of around 85°C, and a heating time period of 10 minutes or more and less than 60 minutes is preferred. The heating treatment of the present invention comprises heat treating a cultured lactic acid bacterial cell in a suspended condition at a certain temperature for a certain time period.
As for the heating means herein, a commonly used means can be used which is not particularly limited. For example, a bacterial cell suspended in a tank may be heated with a heat exchanger. In the present invention, when conducting heat treatment to a lactic acid bacterial cell, it is particularly preferred to subject a cultured bacterial cell to washing treatment by using for example centrifuge or ceramic film to remove medium components, and to conduct heat treatment to a concentrated bacterial cell in a suspension state, in order to obtain an effective heat treatment effect. For the washing treatment using centrifuge or ceramic film, a commonly used centrifuge or filtering system can be used.

### (Antiallergic composition of the present invention)

The antiallergic composition of lactic acid bacterium produced by the present invention has a high antiallergic activity that has been significantly increased and a stable and high antiallergic activity, and can be used effectively by applying in various product forms and in various use. For example, in lactic acid bacteria having undergone heat treatment of the present invention, the activity of the lactic acid bacteria itself has been stopped by the particular heat treatment of the present invention. Therefore, when adding the bacterium to various foods or drinks as an antiallergic composition, it is possible to reduce as much as possible the influence of the bioactivity of the lactic acid bacterium on the original flavor of the foods or drinks. Further, when the antiallergic composition is formulated into various dosage forms, a stable activity can be maintained as an active ingredient.

### (Application of the antiallergic composition produced by the present invention)

The antiallergic composition of lactic acid bacterium produced by the present invention exhibits particularly its effect to environmental allergy including pollen allergy, atopic dermatitis, bronchial asthma, allergic rhinitis and bronchial asthma. The antiallergic composition of lactic acid bacteria produced by the present invention can be used as antiallergic agent after formulation by mixing the active ingredients of the present invention with carrier, excipient, binding agent, diluent and the like that are physiologically acceptable. The antiallergic agents of the present invention can be administered orally or parenterally. As for oral agents, granule, powder medicine, tablets (including sugar-coated tablet), pill, capsule, syrup, emulsion and suspending agent can be exemplified. As for non-oral agents, injection (for example subcutaneous injection, intravenous injection, intramuscular injection, intraperitoneal injection), infusion, external medicine (for example, transnasally administered agents, percutaneous agents, ointments), suppository (for example rectal suppository, vaginal agent) can be exemplified. These formulations can be formulated according to a method commonly used in the field with pharmaceutically acceptable excipients and additives. In the meantime, for formulation, so that the antiallergic composition of lactic acid bacteria produced by the present invention can exert its function appropriately at the right time and effectively *in vivo*, it is preferable to control the time of initiation of elusion, to add the function as agents masking bitterness, or to enhance the stability to oxygen or humidity, for example, as coated materials coated with coating agents having yeast cell wall described in Patent No. 3349677 as main ingredients, or in the form of capsules obtained by capsulizing in soft or hard capsules according to a common procedure, in order to be preferably used in the fields of medicine, health foods and the like. As for excipients or additives pharmaceutically acceptable, carrier, binding agent, flavor, buffer agent, thickening agent, coloring agent, stabilizer, emulsifier, dispersant, suspending agent and preservative can be exemplified. As for pharmaceutically acceptable carriers, magnesium carbonate, magnesium stearate, talc, sugar, lactose, pectin, dextrin, starch, gelatin, tragacanth, methylcellulose, sodium calboxymethylcellulose, low melting wax and cacao butter can be exemplified.

These formulations can be prepared for example as follows. In other words, oral agents can be molded by compressing, by adding for example excipient (for example, lactose, sucrose, starch, mannitol), disintegrator (for example, calcium carbonate, calboxymethylcellulose calcium), binding agent (for example α-starch, gum alabic, calboxymethylcellulose, polyvinylpyrrolidone, hydroxypropyl cellulose) or lubricant (for example talc, magnesium stearate, polyethylene glycol 6000), then by coating according to need, by a publicly known method for the purpose of masking the taste and keeping enteric property and sustained action. As for coating agents, for example, ethyl cellulose, hydroxymethylcellulose, polyoxyethylene glycol, cellulose acetate phtalate, hydroxypropylmethylcellulose phtalate and eudragit (Rhom, Germany; methacrylate/acrylate copolymer) can be used.

Injections can be prepared by dissolving, suspending or emulsifying the active ingredients with dispersant (for example, Tween 80 (Atlas Powder, U.S.A), HCO 60 (Nikko Chemicals), polyethylene glycol, carboxymethylcellulose, sodium alginate), perservative (for example, methyl paraben, propyl paraben, benzyl alcohol, chlorobutanol, phenol), isotonization agent (for example, sodium chloride, glycerin, sorbitol, glucose, invert sugar) and the like to aqueous solvent (for example, distilled water, physiological saline, Ringer solution and the like) or oleaginous solvent (vegetable oil such as olive oil, sesame oil, cotton seed oil and corn oil, propylene glycol), and the like. At that time, additives such as disintegration adjuvant (for example sodium salicylate, sodium acetate), stabilizer (for example human serum albumin) and soothing agent (for example, benzalkonium chloride, hydrochloric procaine) can be added if desired.

External agents can be prepared by making the active ingredients to a composition in a solid, semisolid or liquid form. For example, the composition in a solid form mentioned above can be prepared by the active ingredient itself or by adding and mixing excipients (for example lactose, mannitol, starch, microcrystalline cellulose, sucrose), thickening agents (for example, natural gums, cellulose derivative, acrylic acid polymer) and the like to make in a powder form. The composition in a liquid form mentioned above can be prepared almost with the same manner as the injection. As for the compositions in a semisolid form, aqueous or oleaginous gel agents or ointments are preferred. Moreover, all of these compositions can comprise pH controller (for example, carbonic acid, phosphoric acid, citric acid, hydrochloric acid, sodium hydroxide), perservative (for example, paraoxy benzoic acid esters, chlorobutanol, benzalkonium chloride) and the like. Suppositories can be formulated by making the active ingredients to an aqueous or oleaginous composition in a solid, semisolid or liquid form. As for oleaginous base used for the compositions, glyceride of higher fatty acid (for example, cacao oil, witepsols (Dynamit Nobel), medium fatty acid (for example, Migliores (Dynamit Nobel)) or vegetable oils (for example, sesame oil, soybean oil, cotton seed oil) can be exemplified. As for aqueous base, polyethylene glycols and propylene glycol can be exemplified. Moreover, as for aqueous gel base, natural gums, cellulose derivative, vinyl polymer and acrylic acid polymer can be exemplified.

### (Use by compounding into foods or drinks)

The antiallergic composition of lactic acid bacteria produced by the present invention can be used as foods or drinks with antiallergic function by compounding into foods or drinks. To use the antiallergic composition of lactic acid bacteria produced by the present invention by compounding into foods or drinks, the effective dose of the active ingredients is added and compound during the stage of manufacturing raw material or after the product is manufactured and the like. Here, the term "effective dose of the active ingredients" relates to the content wherein the active ingredients are ingested within the following range, when the amount generally consumed for each food and drink is ingested.

In other words, as for the dosage or intake of the effective dose of the active ingredients of the present invention to foods or drinks, it can be determined depending on the recipient, the age and body weight of the recipient, symptoms, administered time, dosage form, administering method, the combination of agents and the like. For example, when the active ingredients of the present invention are administered as medicine orally, it can be administered 1 to 3 times per day within the range of: 0.1 - 100 mg/kg body weight (preferably 1-10 mg/kg body weight) when administered orally, and 0.01 - 10 mg/kg body weight (preferably 0.1 - 1 mg/kg body weight) when administered parenterally. The agents having other acting mechanisms used by combining with the active ingredients of the present invention can be also determined appropriately by using the dosage used clinically as standard. When the dosage or intake of the effective dose of the active ingredients of the present invention to foods or drinks is indicated by the number of the lactic acid bacteria, it is preferable that the intake is 5 × 10⁹ or more per day, more preferably 1 × 10¹⁰ or more per day, most preferably 5 × 10¹⁰ or more per day. Therefore, the number of the lactic acid bacteria to be contained per each food is determined according to the amount of foods or drinks generally ingested per day.

In the present invention, the active ingredients of the antiallergic composition of lactic acid bacteria produced by the present invention can be compounded by itself or in a form of formulation described above to foods or drinks. More concretely, the foods or drinks of the present invention can take various forms of usage, by compounding the active ingredients of the present invention with base materials appropriately, and prepare as foods or drinks by itself, or by further compounding various proteins, sugars, fats, trace elements, vitamins and the like, or prepared in a liquid, semi-liquid or solid form, or further added or compounded to general foods or drinks, or the like.

As for the field of foods or drinks using the lactic acid bacteria, it is roughly classified into dairy products, meats, breads, beverages and vegetables, based on examples of its utilization, heretofore. In case of these foods or drinks using lactic acid bacteria, it is possible to confer antiallergic function to the foods or drinks by using the lactic acid bacteria with a high antiallergic activity of the present invention as an additive for the manufactured foods or drinks.

The foods of the present invention can be prepared as health food, functional food, specified health food, or patient food wherein antiallergic function is added. Moreover, it is not particularly limited to form of food, and it can be in a form of beverage wherein antiallergic function is added. As the active ingredients of the present invention have antiallergic activity, it is possible to provide foods that are possible to ingest continuously and having function of preventing the development of allergy or treating allergy, by compounding the active ingredients of the present invention to foods ingested daily or health foods or functional foods and the like ingested as supplements. By using the lactic acid bacteria with high antiallergic activity of the present invention by compounding in foods or drinks that do not contain ingredients inducing allergy in the foods or drinks, such as tea drinks, health drinks or tablets, it is possible to provide foods or drinks with antiallergic function completely blocked from allergic problems.

### (Use in formulation form of foods or drinks)

In the present invention, as for health foods and functional foods compounding the lactic acid bacteria with high antiallergical activity produced by the present invention, various products can be exemplified, and as for the production of these health foods and functional foods, besides food materials and food additives generally used, it can be used in formulation form of foods or drinks using adjuvants such as excipients, extender, binding agent, disintegrator, lubricant, dispersant, preservative, wetting agent, solving adjuvant, antiseptic, stabilizer and capsule. Examples of the adjuvants include: lactose, fructose, glucose, starch, gelatin, magnesium carbonate, synthetic magnesium silicate, talc, magnesium stearate, calcium carbonate, methylcellulose, carboxymethylcellulose, or salt thereof, gum alabic, polyethylene glycol, syrup, vaserine, glycerin, ethanol, propylene glycol, citric acid, sodium chloride, sodium sulfite, sodium phosphate, pullulan, carrageenan, dextrin, reduced palatinose, sorbitol, xylitol, stevia, artificial sweetener, citric acid, ascorbic acid, acidulant, sodium bicarbonate, sucrose ester, vegetable hydrogeneted oil, potassium chloride, safflower oil, bees wax, soybean lecithin and flavor. As for the production of such health foods and functional foods, it can be referred to reference books on drug formulation, for example "Practical guide of Japanese Pharmacopoeia (General rule on formulation)" (Hirokawa Shoten).

In the present invention, as for forms particularly suitable for health foods and functional foods, form of tablet, capsule, granule, powder medicine, suspension and emulsion can be exemplified, and from the point of view of the object that the present invention is aiming to solve, it is preferred that health foods and functional foods compounding the lactic acid bacteria with high allergic activity of the present invention are combining raw materials that do not comprise raw material specific to allergy.

Specific examples of methods for producing health foods and functional foods in the form of tablets include a producing method wherein the mixture compounding the lactic acid bacteria with high antiallergic activity produced by the present invention is compressed to a certain form, or a method wherein the mixture being wet with solvent such as water or alcohol is formed in a certain form or poured into a certain mold. Specific examples of methods for producing health foods and functional foods in the form of capsules include a method of filling capsules wherein the formulation compounding the lactic acid bacteria with high antiallergic activity of the present invention is filled into capsules in form of liquid, suspension, paste, powder or granule, or a producing method by encapsulating and forming with capsule base materials, such as hard capsules and soft capsules.

### (Compounding to foods)

Moreover, in the present invention, it is possible to prepare foods with antiallergic function by compounding the lactic acid bacteria with high antiallergic activity produced by the present invention to foods. Examples of these foods or drinks include: cakes such as cream caramel, cookie, cracker, potato chips, biscuit, bread, cake, chocolate, donuts and jelly; Japanese cakes such as rice cracker, faded black, daifuku (rice cake filled with sweet jam paste), been cake and other steamed bean-jam bum, sponge cake; breads and snacks such as cold dessert (candy and the like) and chewing gum; noodles such as wheat noodle, buckwheat noodle and kishimen (flat wheat noodle); fish cakes such as steamed fish paste, ham and fish meat sausage; meat products such as ham, sausage, hamburger and canned beef; seasonings such as salt, pepper, soybean paste (miso), soybean sauce, sauce, dressing, mayonnaise, ketchup, sweetener and pungent seasonings; grilled foods such as akashiyaki(soft octopus ball), takoyaki (octopus ball), monjayaki (doughy crape-like pancake), okonomiyaki (savory pancake), fried noodles and fried wheat noodles; dairy products such as cheese and hard type yogurt; various prepared foods such as fermented soybeans, pressed tofu, tofu, yam paste, rice dumpling, pickles, fish boiled in soy sauce, kop-zi, shao mai, croquette, sandwich, pizza, hamburger and salad; various powders (meat products such as beef, pork and chicken; fishery products such as shrimp, scallop, freshwater clam and dried tangle; vegetables and fruits, plants, yeast and algae); powdered solid products of fat and flavoring ingredients (vanilla, citrus, bonito and the like); and powdered foods or drinks (instant coffee, instant tea, instant milk, instant soup, miso soup and the like), but it is not limited to these.

### (Compounding to beverages)

As for the composition with high antiallergic activity of lactic acid bacteria produced by the present invention, particularly by using it in form of beverage, it is possible to provide a beverage with antiallergic function that can be ingested everyday continuously, with antiallergic function that becomes effective with the amount possible to ingest continuously. When compounding the lactic acid bacteria with high antiallergic activity produced by the present invention to beverage, the content of the lactic acid bacteria can be determined appropriately, but generally the amount to be compound is applied such that the antiallergic activity of the lactic acid bacteria can be effective in an ingestible amount continuously as beverage is applied. When the dosage or intake of the effective dose of the active ingredients of the present invention to foods or drinks are expressed by the number of the lactic acid bacteria, it is preferable that the intake is 5 × 10⁹ or more cells per day, more preferably 1 × 10¹⁰ or more cells per day, most preferably 5 × 10¹⁰ or more cells per day. Therefore, the number of the lactic acid bacteria strain to be contained per each beverage is determined, with the index mentioned above, according to the amount of drinks generally ingested per day. For example, if 100 g of beverage is ingested per day, it is preferable to add 10⁹ or more of bacteria per 100 g of beverage. On the other hand, when considering a range that does not to damage the flavor or the appearance of the beverage by adding the lactic acid bacteria, 10¹¹ or less cells is preferred. Moreover, 5 × 10¹⁰ or less cells is more preferred. Therefore, as for the concentration of the lactic acid bacteria having high antiallergic function, and being stabilized, having good taste and good storage ability, it is most preferred to be within 10⁹-10¹¹ cells per 100 g of beverage. Meanwhile, as for the relationship between the number of the lactic acid bacteria and the weight of dried bacteria, for example, for *L. paracasei* KW3110 strain, the number of strain 10¹² bacteria corresponds to 1 g weight of dried strain.

In the present invention, as for beverage compounding the lactic acid bacteria with high antiallergic activity produced by the present invention, various beverages can be exemplified, and for manufacturing these, any of saccharide, flavor, juice, food additives and the like used for general beverage formulation can be used. As for manufacturing beverages, it can be referred to existing reference books, for example "Revised new edition: soft drinks" (Kohrin).

Specific examples of beverages to compound include: alcoholic beverage (whiskey, bourbon, spirit, liqueur, wine, fruit wine, rice wine (sake), Chinese wine, distilled spirit, beer, non-alcohol beer with 1% or less of alcohol proof, law-malt beer, chuhai (carbonated distilled spirit) and the like), or non-alcoholic beverage (drink type yogurt, juice of apple, orange, grape, banana, pear, plum, watermelon and the like, vegetable juice of tomato, carrot, celery, cucumber an the like, soft drink, milk, soy milk, coffee, cocoa, various herb tea such as red tea, green tea, barley tea, brown rice tea, natural leaf tea, refined green tea, roasted green tea, oolong tea, curcuma tea, black tea, Rooibos tea, rose tea, chrysanthemum tea, mint tea and jasmine tea, sport drink, mineral water, energy drink and the like).

By exemplifying by categories of beverage, tea drinks such as green tea, oolong tea, red tea, barley tea, blended tea, and coffee, beverage containing juice, vegetable juice, sport drink, energy drink can be exemplified.

In the present invention, when manufacturing beverages with antiallergic function, it is possible to sterilize beverage appropriately, according to methods defined by Food Sanitation Law. As for the sterilizing method, it is possible to use Pasteur Sterilization, hot pack sterilization, UHT sterilization, retort sterilization and the like, based on pH of the beverage.

Moreover, as for the form of the product, the form of beverage in a sealed container used generally as product form of beverages is particularly preferred, and as for the sealed container, any form of can, bottle, PET bottle and paper container can be used. Moreover, there is no particular limitation for the volume, and it can be determined generally by considering the amount that a consumer ingests daily, the number of the lactic acid bacteria to be compounded, and the number of bacteria necessary daily.

The present invention will be explained in detail in the following, but the present invention will not be limited by these.

### Example 1

### <1. Method for measuring antiallergic activity>

The antiallergic activity of lactic acid bacteria *in vitro* was measured by measuring IL-12 levels released in the medium when culturing the bacterium in combination with mouse spleen lymphocytes. Seven to ten weeks-old BALB/c mice (Charles River) were intraperitoneally injected with 1 mg of ovalbumin (OVA) at day 0 and day 6 with 2 mg of aluminum hydroxide being an adjuvant. The animals were dissected on day 13, to isolate spleen and to prepare lymphocytes. Spleen lymphocytes were suspended in RPMI 1640 (SIGMA) medium supplemented with FCS (Rosche) and OVA so that the final concentrations become 10% and 1 mg/ml, respectively in order to obtain a cell concentration of 2.5 × 10⁶ cells/ml. Then, lactic acid bacteria were added to the above medium to obtain 0.25 µg/ml, and cultured for 1 week at 37°C with a CO₂ concentration of 5%. The cultured supernatant was recovered by centrifugation, and IL-12 was measured by using OptEIA ELISA (Becton Dickinson).

### (Indication of experiment results)

IL-12 production levels of the sample were compared with those of the control, *L. paracasei* KW3110 strain, and the relative levels are shown.

### (Preparation of the control bacterium)

Bacteria which have undergone static culture using MRS medium at 37°C for 48 hours were washed 3 times with sterilized water, suspended in the sterilized water, and then treated at 100°C for 30 minutes for sterilization. The resultant was lyophilized and suspended in PBS.

### <2. Preparation of the sample L. paracasei KW3110 strain: culture>

A medium containing similar level of nitrogen source, carbon source and inorganic materials as MRS medium was put in a 50 L-tank, and was steam-pasteurized at 120°C for 20 minutes. To this, lactic acid bacterial cell proliferated appropriately in MRS medium were added, and cultured at 32°C, 60 rpm, by adjusting pH to 5.5 with sodium hydroxide, for 48 hours. The prepared culture solution was taken, washed by centrifugation to obtain bacterial cell suspension.

### <3. Heat treatment>

The bacterial cell suspension obtained in the above 2. was heated in a warm bath adjusted to each temperature, and samples were recovered at each time period, when the bacterial cell suspension attained each temperature. The recovered samples were lyophilized, and subjected to the IL-12 production activity evaluation.

### <4. Experiment results>

The above experiment results are shown in Fig. 1 and Table 1. In the present experiment, experiments were repeated once for the test group at 85°C, twice for the test group at 60°C, and 3 times for other test groups, and the average levels are shown. "Non-heated" denotes bacteria that have been lyophilized after washing the culture solution.
Fig. 1 shows the ratio of IL-12 production level (ratio with respect to the control) for each heating condition. Table 1 shows the IL-12 production activity level (relative level with respect to control (%)) for each heating condition.

**[Table 1]**

| Preparation method of the sample | | Average level | Standard deviation |
|---|---|---|---|
| | Non-heated | 11.33 | 9.36 |
| 40°C | 10 min | 27.25 | 6.95 |
| | 30 min | 23.97 | 4.09 |
| | 60 min | 20.42 | 4.02 |
| 50°C | 10 min | 20.70 | 3.06 |
| | 30 min | 17.38 | 4.16 |
| | 60 min | 22.84 | 10.05 |
| 60°C | 10 min | 115.25 | 9.82 |
| | 30 min | 126.66 | 0.69 |
| | 60 min | 132.32 | 28.94 |
| 70°C | 10 min | 130.16 | 11.38 |
| | 30 min | 130.72 | 11.91 |
| | 60 min | 134.46 | 15.35 |
| 85°C | 10 min | 144.45 | |
| | 30 min | 140.81 | |
| | 60 min | 149.88 | |
| 100°C | 10 min | 140.64 | 18.07 |
| | 30 min | 127.42 | 7.89 |
| | 60 min | 111.00 | 8.24 |

### Industrial Applicability

According to the present invention, it is possible to provide a lactic acid bacterium which antiallergic activity has been significantly increased, for a lactic acid bacterium with an excellent antiallergic activity. Further, with a particular heat treatment of the present invention, it is possible to provide a lactic acid bacterial cell having a stable and high antiallergic activity by stopping the activity of the lactic acid bacterium itself, and preventing as much as possible the denaturation of the bacterial cell itself by heat treatment. Therefore, according to the present invention, an antiallergic composition comprising a stable and high antiallergic composition as active ingredient by applying to various product forms and in various use, that can be used effectively is provided.

## Claims

1. A method for producing a heat treated bacterial cell of *Lactobacillus paracasei* KW3110 strain or its mutant strain having a high antiallergic activity, wherein a heat treatment is conducted to *Lactobacillus paracasei* KW3110 strain or its mutant strain at a temperature of 60°C or more and less than 100°C, for a time period of 10 minutes or more and less than 60 minutes.

2. The method for producing a heat treated bacterial cell of *Lactobacillus paracasei* KW3110 strain or its mutant strain having a high antiallergic activity according to claim 1, wherein the heat treatment to *Lactobacillus paracasei* KW3110 strain or its mutant strain is conducted after culturing *Lactobacillus paracasei* KW3110 strain or its mutant strain, and removing a medium component by conducting washing treatment to the cultured bacterial cell.

3. A heat treated bacterial cell of *Lactobacillus paracasei* KW3110 strain or its mutant strain produced by the production method of claim 1 or 2.

4. An antiallergic composition comprising the heat treated bacterial cell of *Lactobacillus paracasei* KW3110 strain or its mutant strain produced by the production method of claim 1 or 2 as an active ingredient.
